# EUROPEAN PATENT APPLICATION

(11) **EP 2 641 566 A1**
(43) Date of publication of application: **25.09.2013**
(21) Application number: 13172481.7
(22) Date of filing: 30.06.2009
(51) Int. Cl.: A61F 2/12

(54) **Fillable prosthetic implant with gel-like properties**

(30) Priority: 30.06.2008 US 76818 P
(62) Divisional of application: 09774275.3
(71) Applicant: ALLERGAN, INC., Irvine CA 92612 (US)
(72) Inventor: Powell, Thomas E., Santa Barbara, CA California 93111 (US)
(74) Representative: HOFFMANN EITLE

(57) **Abstract**

The present invention provides a soft prosthetic implant (10) comprising a flexible shell (30) having a shell wall defining an inner chamber (34), and a volume of dry powder (40) whose particles have diameters in the range of 10⁻⁹ to 10⁻⁶ m within the inner chamber, the dry powder being capable of forming a gel (50) when mixed with a fluid; characterised in that the dry powder comprises at least one of 2-hydroxyethyl methacrylate, 2-hydroxypropyl methacrylate and poly(2-hydroxyethyl methacrylate). A method for producing a soft prosthetic implant is also provided.

## Description

### Field of the Invention

The present invention relates to soft prosthetic implants and, more particularly, to other than gel-filled prostheses with gel-filled characteristics.

### Backgtound of the Invention

Implantable prostheses are commonly used to replace or augment body tissue. In the case of breast cancer, it is sometimes necessary to remove some or all of the mammary gland and surrounding tissue that creates a void that can be filled with an implantable prosthesis. The implant serves to support surrounding tissue and to maintain the appearance of the body. The restoration of the normal appearance of the body has an extremely beneficial psychological effect on post-operative patients, eliminating much of the shock and depression that often follows extensive surgical procedures. Implantable prostheses are also used more generally for restoring the normal appearance of soft tissue in various areas of the body, such as the buttocks, chin, calf, etc.

Soft implantable prostheses typically include a relatively thin and quite flexible envelope or shell made of vulcanized (cured) silicone elastomer. The shell is filled either with a silicone gel or with a normal saline solution. The filling of the shell takes place before or after the shell is inserted through an incision. The present invention pertains to a fluid-filled prosthesis that is typically filled after implant.

Gel-filled breast implants have been in use for over 40 years. In the 1960s, the implants were filled with a relatively thick, viscous silicone gel which created a somewhat non-responsive, unnatural feel. During the 1970s and into the 1980s, a softer, more responsive silicone gel was introduced. Since the 1980s up to the present, improvements to the silicone gel has rendered them somewhat more cohesive and firm without being non-responsive.

Medical prostheses from a safety standpoint should be chemically inert, noninflammatory, nonallergenic, and noncarcinogenic. In the case of breast implants, the prosthesis ideally should also simulate the viscoelastic properties of the normal human breast, and be radiolucent to mammography. It is further important that breast implants create a natural "feel" and desirable aesthetics. Although silicone gels are considered by many physicians to be the best choice for meeting all these requirements, some consumers remain wary of the safety of silicone gels.

Perhaps unjustifiably, some consumers may consider saline-filled implants to be safer than silicone gel-filled implants. Further, unlike silicone gel filled implants which are implanted fully intact and filled, saline implants are usually implanted in the breast as an empty shell or a partially filled shell, and then are inflated to their final size after implantation. For this reason, a smaller incision may be required for implantation of a saline-filled implant relative to a silicone gel-filled implant. Another advantage to saline-filled implants is that physician may adjust the volume of a saline-filled implant by adding or removing saline, for example, with a syringe, after the implant has been positioned in the breast. Unfortunately, saline lacks the viscoelastic properties of silicone gel and consequently, saline-filled implants generally have a less natural feel and appearance.

Despite many advances in the construction of breast implants, there remains a need for a breast implant that provides the natural feel of a silicone gel-filled prosthesis with the perceived safety and advantages of a saline-filied prosthesis.

### Summary of the Invention

The present invention provides a soft prosthetic breast implant comprising a flexible shell and a quantity of biologically inert, dry hydrogel particles, preferably in nanoparticle form, within an inner chamber of the shell. Upon the addition of a liquid for example, an aqueous medium, to the dry hydrogel in the chamber, for example, after implantation of the prosthesis in a human body, a hydrogel-filled implant is formed in vivo.

### Brief Description of the Drawings

Features and advantages of the present invention will become appreciated and the same may be better understood with reference to the specification, claims, and appended drawings wherein:

Figure 1 is a schematic view of a torso of a breast implant patient showing several locations for implant incisions;

Figure 2 is a sectional view through a breast implant of the invention positioned within a breast and being filled with a fluid;

Figure 3 is a schematic view of a torso of a breast implant patient shown after implantation and filling of two breast implants of the present invention;

Figure 4 is a cross-sectional view through an uninflated implant of the present invention having a quantity of dry nanoparticles therein; and

Figure 5 is a cross-sectional view through the breast implant of Figure 4 after having been inflated with a fluid to mix with the dry nanoparticles and form a gel.

### Detailed Description

A gel is a three-dimensional polymeric network that has absorbed a liquid to form a stable, usually soft and pliable, composition having a non-zero shear modulus. When the liquid absorbed by a gel is water, the gel is called a hydrogel. A hydrogel is a three dimensional polymeric structure that itself is insoluble in water but which is capable of absorbing and retaining large quantities of water to form a stable, often soft and pliable structure. A hydrogel may contain over 99% water. Many hydrogel-forming polymers are biologically inert. For these reasons, it has been recognized that hydrogels are particularly useful in a wide variety of biomedical applications.

A particulate substance or particulate material in the context of the present invention is a substance in loose, particulate form, for example, in powder form. The particles may be uniform in size and/or shape, though some variation is acceptable. Dry nanoparticles means that the nanoparticles are in solid form with no liquid in the interstitial spaces. Of course in any solid there may be some H₂O, more in humid conditions, and the term "dry" should not be construed to mean completely dehydrated.

Particulate gels for purposes of the present invention can be formed by a number of procedures such as direct or inverse emulsion polymerization, or they can be created from bulk gels by drying the gel and then grinding the resulting xerogel to particles of a desired size. The particles can then be re-solvated by addition of a fluid medium. Particles having sizes in the micrometer (µm, 10⁻⁶ m) to nanometer (nm, 10⁻⁹ m) diameter range can be produced by this means. Depending on the definition, a nanoparticle is a microscopic particle whose size is measured in nanometers (nm), for example, a particle having at least one dimension less than 200 nm, or by some accounts a size less than 100 nm (10⁻⁷ m). Such nanoparticles have strikingly different properties relative to larger sized particles, and these properties are found useful in many applications. It has been recognized that the properties of materials change as their particle size approaches the nanoscale. The interesting and sometimes unexpected properties of nanoparticles may be in part due to the aspects of the surface of the material dominating the properties in lieu of the bulk properties. The percentage of atoms at the surface of a material becomes significant as the size of that material approaches the nanoscale.

In one aspect of the invention, a soft implant is provided wherein the implant comprises a flexible shell having a shell wall defining an inner chamber, and a quantity of dry hydrogel nanoparticles, within the inner chamber.

In another aspect of the invention, a method is provided for forming a soft implant wherein the method generally comprises the steps of molding a flexible implant shell having a shell wall defining an inner chamber, the inner chamber having a predetermined volume when inflated, and introducing into the inner chamber a dry volume of dry nanoparticles equal to between about 1% to about 30%, about 40% or about 50% of the predetermined volume.

In yet another aspect of the invention, a method of implanting a soft implant or prosthesis is provided wherein the method generally comprises the steps of providing a flexible shell having a shell wall defining an inner chamber therein and a quantity of dry nanoparticles within the inner chamber, inserting the flexible implant shell into a body, and introducing a fluid into the inner chamber to combine with the dry nanoparticles and form a gel in vivo.

Preferably, the dry particles in the shell chamber in accordance with the invention comprise dry hydrogel nanoparticles. Such nanoparticles are known to aggregate when combined or missed with a suitable medium, for example an aqueous fluid, to form an aggregated hydrogel having the advantageous properties of a nanoparticle aggregate. As used herein, the term "aggregate" refers to a bulk material composed of a plurality of hydrogel particles held together by inter-particle and particle-liquid forces, such as, without limitation, hydrogen bonds. More detail on nanoparticles and proposed uses therefor can be found in U.S. Patent No. 7,351,430, and U.S. Patent Publication No. 2008/0063716, filed October 30, 2007, the entire disclosure of each of which being expressly incorporated herein by reference.

One source of materials for use in the present invention is Uluru, Inc. of Addison TX. Uluru, Inc. has developed a biocompatible material which utilizes suspensions containing hydrogel nanoparticles that, when aggregated, form a bulk gel material of varying strength and/or elasticity.

The dry hydrogels used in the present invention are preferably hydroxyl-terminated methacrylate monomers (2-hydroxyethylmethacrylate and 2-hydroxypropylmethacrylate), or pHEMA (poly-2-hydroxyethyl methacrylate).

Moro ct al. U.S. Patent Application Publication No. US 2008/0063716. proposes formation of aggregates in the body by injecting a suspension containing hydrogel nanoparticles into the body. As described by Moro et al., when the suspension of particles is injected into the body, the absolute zeta potential of the particles is lowered and the particles self-assemble into a compact elastic, shape-retentive aggregate. Moro et al. describes that aggregate assumes and retains the shape of the region of the body into which it is injected.

In some embodiments of the present invention, hydrogel materials such as those proposed by Moro et al. are used, though in a different form and delivery technique than heretofore suggested. In accordance with the present invention, instead of directly injecting a suspension of hydrogel nanoparticles into a body, the bulk polymer is first desiccated, or converted to dry nanoparticles, preferably a fine powder. This substance is then introduced to the inner chamber of a fillable implant shell, which can then be stored for extended periods without degradation or significant degradation before use. Ultimately, a surgeon inserts the implant shell including the dry particles into a body cavity, and then fills or inflates the shell with an injectable medium, for example, a liquid, for example, saline. The medium will combine with the particles to form a colloidal suspension in the shell, resulting in a gel, preferably a hydrogel.

In a particularly preferred embodiment, the dry nanoparticles comprise hydroxyl-terminated methacrylate monomers (2-hydroxyethylmethacrylate and 2-hydroxypropylmethacrylate), or pHEMA (poly-2-hydroxyethyl methacrylate), in a quantity sufficient such that when mixed with fluid medium the mixture forms a desired volume of a gel within the implant shell.

The fluid medium used to mix with the dry nanoparticles is preferably an inert aqueous composition, for example, saline or pure water, or other suitable liquid that will cause the dry particles to coalesce and form a gel. It is contemplated that in some embodiments of the invention, an oil may be used as the fluid medium. Fallot, et al., U.S. Patent No. 6,156,066, the entire disclosure of which is incorporated herein by this reference, discloses the advantages of using various oils in implants. For example, oils are lighter in weight than water, many having an atomic weight of 6 or less, and are X-ray translucent, thus being advantageous in mammography procedures. Indeed, many other fluids which have been proposed for use as fillers for soft implants many be useful in the context of the present invention, and the fluid medium should not be considered limited to any particular fluid unless otherwise stated.

The dry nanoparticles may be prepared by first treating the hydrogel nanoparticles to remove residual monomer and any other undesirable materials before being dried. Drying may be through freeze-drying or other known technique. The prepared or commercial dry, brittle bulk polymer is then broken up by grinding, micro-pulverizing and the like and the fragments are sieved using techniques known in the art to separate particles of different size.

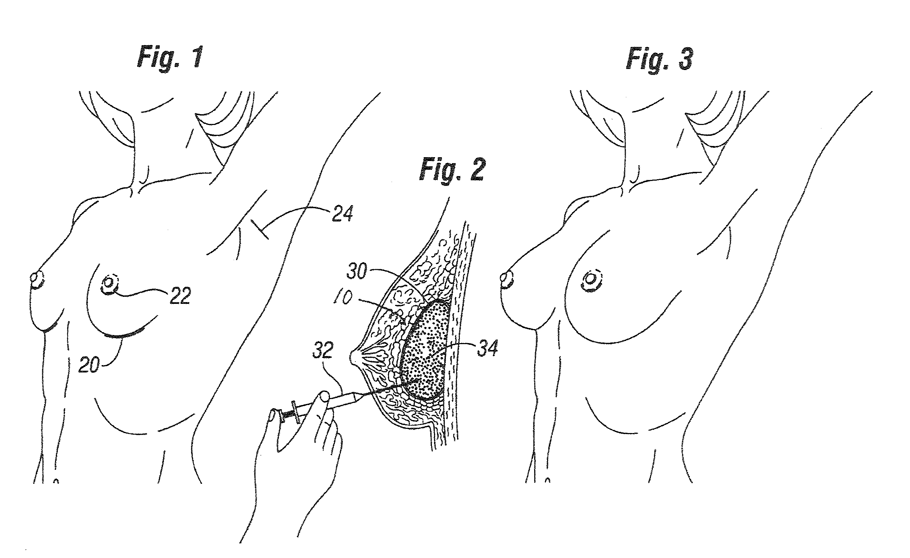

Figures 1-3 illustrate use of the breast implant filling technique of the present invention. In Figure 1, the torso of a breast implant patient is shown with number of possible incisions used by surgeons. Specifically, the possible incisions include an inframammary incision 20, a periareolar incision 22, and a transaxillary incision 24. It should be understood that the breast implants of the present invention may be delivered through any of these incisions, or others, depending on the preference of the surgeon after consultation with the patient.

Figure 2 shows a cross-section of a breast having implanted therein an implant shell 10 of the present invention. The shell 10 comprises a flexible, preferably elastomeric, member 30 having a shell wall defining an inner chamber 34. The shell further comprises a quantity of dry particles, for example, dry hydrogel nanoparticles, located within the inner chamber. After the shell has been implanted into the breast, a a syringe 32 or other means is used to deliver a suitable amount of a fluid, for example, an aqueous solution, for example, a saline solution, to the inner chamber 34. The fluid mixes with and hydrates the quantity of dry nanoparticles to form, in vivo, a hydrogel-filled implant.

It can be appreciated that, advantageously, the incision used in the methods of the present invention can be substantially smaller than that required for implanting a conventional pre-filled silicone gel-filled implant. Furthermore, the resulting prosthesis is more natural in appearance and feel than a typical saline-filled implant. Figure 3 shows the exterior appearance of the breasts after the implant procedure.

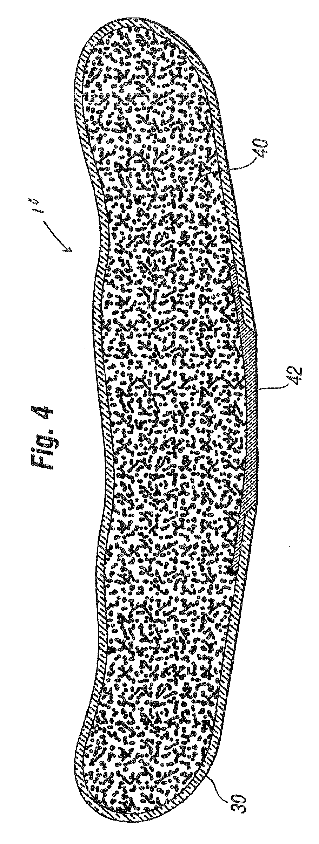

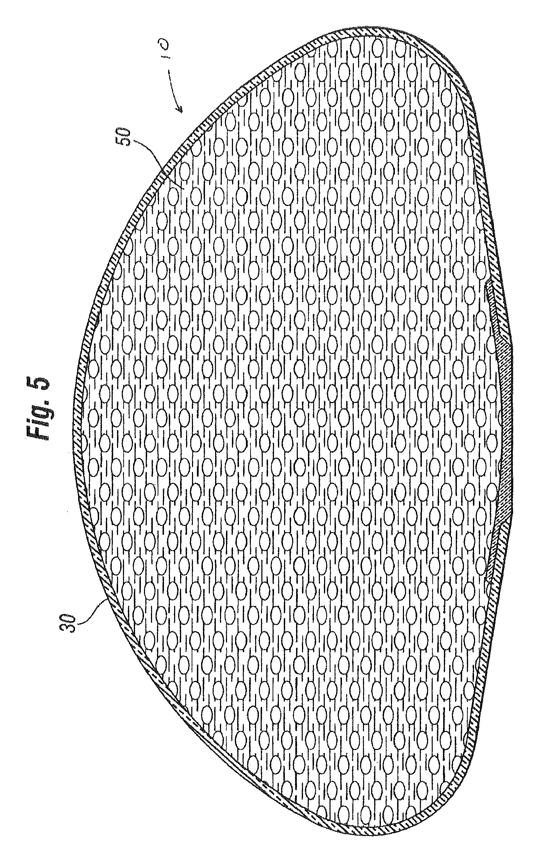

Figure 4 is a cross-sectional view through the shell 10 prior to hydration of the dry material 40. A common material for the shell wall is a vulcanized (cured) silicone elastomer. Fig. 5 is a cross-sectional view through the shell 10 after hydration of the material, which has formed a nanoparticle gel 50. In one aspect of the invention, the flexible member 30 defines an inner chamber having a first volume defined by the volume of dry particulate material 40 and a second volume greater than the first volume when the dry particulate material is hydrated (e.g. volume af gel 50) as shown in Fig. 5.

For example, the shell 30 defines an inner chamber having a predetermined volume when inflated with a fluid greater than the first volume when not inflated with the fluid. The shell 30 is considered inflated when the inner chamber is full of a fluid at ambient pressure. Preferably, the quantity of dry nanoparticles has a dry volume of between about 1% to about 30%, about 40% or about 50% of the predetermined volume of the inflated shell. In a specific embodiment, the quantity of dry nanoparticles has a dry volume of about 10% of the predetermined volume of the shell.

The shell 30 may be formed using a variety of techniques, such as dip-molding or rotational molding. In order to introduce the dry nanoparticles after formation of the elastomeric shell, a fill aperture will be left that is covered with a patch 42, as is known in the art. The patch 42 may be self-sealing, or the entire shell 30 may be self-sealing, to permit puncture by a fill needle after implant without risk of leakage.

Although the invention has been described and illustrated with a certain degree of particularity, it is understood that the present disclosure has been made only by way of example, and that numerous changes in the combination and arrangement of parts can be resorted to by those skilled in the art without departing from the scope of the invention, as hereinafter claimed.

Embodiments of the invention:
1. A shell for a soft prosthetic implant, comprising:
   a flexible member having a shell wall defining an inner chamber; and
   a volume of dry nanoparticle material within the inner chamber that forms a gel when mixed with a fluid.
2. The shell of 1 wherein the dry nanoparticle material comprises dessicated hydrogel nanoparticles.
3. The shell of 1 wherein the flexible member is elastomeric.
4. The shell of 1 wherein the flexible member defines an inner chamber having a first volume defined by the volume of dry nanoparticle material and a second volume greater than the first volume when the dry nanoparticle material is hydrated.
5. The shell of 4 wherein the first volume is about 1% to about 60% of the second volume.
6. The shell of 4 wherein the first volume is about 10% of the second volume.
7. A method of forming a soft prosthetic implant shell, comprising:
   molding a flexible implant member having a shell wall defining an inner chamber, the inner chamber having a predetermined volume when inflated; and
   introducing into the inner chamber a volume of dry nanoparticle material equal to between about 1 to about20% of the predetermined volume.
8. The method of 7 further comprising the step of sealing the flexible implant shell after the step of introducing a volume of dry nanoparticle material.
9. A method of implanting a soft prosthesis, comprising:
   providing a flexible implant shell having a shell wall defining an inner chamber therein, and a quantity of dry nanoparticle material within the inner chamber;
   inserting the flexible implant shell into a body cavity; and
   introducing a fluid into the inner chamber to mix with the dry nanoparticle material and form a gel in vivo.

## Claims

1. A soft prosthetic implant (10) comprising:
a flexible shell (30) having a shell wall defining an inner chamber (34); and
a volume of dry powder (40) whose particles have diameters in the range of 10⁻⁹ to 10⁻⁶ m within the inner chamber, the dry powder being capable of forming a gel (50) when mixed with a fluid;
**characterised in that** the dry powder comprises at least one of 2-hydroxyethyl methacrylate, 2-hydroxypropyl methacrylate and poly(2-hydroxyethyl methacrylate).

2. The implant of Claim 1, wherein the dry powder (40) comprises dessicated hydrogel nanoparticles.

3. The implant of Claim 1, wherein the flexible shell (30) is elastomeric.

4. The implant of Claim 1, wherein the flexible shell (30) defines an inner chamber (34) having a first volume defined by the volume of the dry powder (40) and a second volume when the dry powder is hydrated, the second volume being greater than the first volume.

5. The implant of Claim 4, wherein the first volume is 1-60% of the second volume.

6. The implant of Claim 5, wherein the first volume is 10% of the second volume.

7. A method for producing a soft prosthetic implant (10) comprising:
moulding a flexible implant shell (30) having a shell wall which defines an inner chamber (34) having a predetermined volume when inflated; and
introducing a dry powder (40) whose particles have diameters in the range of 10⁻⁹ to 10⁻⁶ m into the inner chamber in a volume equal to 1-20% of the predetermined volume;
**characterised in that** the dry powder comprises at least one of 2-hydroxyethyl methacrylate, 2-hydroxypropyl methacrylate and poly(2-hydroxyethyl methacrylate).

8. The method of Claim 7, further comprising the step of sealing the flexible implant shell (30) after introducing the dry powder (40) into the inner chamber (34).
